(19) Europäisches Patentamt

European Patent Office

Office européen des brevets

(11) **EP 0 858 791 B1**

(12) **EUROPEAN PATENT SPECIFICATION**

(45) Date of publication and mention
of the grant of the patent:
**21.08.2002 Bulletin 2002/34**

(51) Int Cl.⁷: **A61F 13/15**

(21) Application number: **98101917.7**

(22) Date of filing: **04.02.1998**

(54) **Absorbent article**

Absorbierender Artikel

Article absorbant

(84) Designated Contracting States:
**DE FR GB**

(30) Priority: **06.02.1997 JP 2415797**

(43) Date of publication of application:
**19.08.1998 Bulletin 1998/34**

(73) Proprietor: **KAO CORPORATION**
**Chuo-ku, Tokyo (JP)**

(72) Inventors:
 • **Kameo, Yoji, c/o Kao corp. Research Lab.**
 **Tochigi-ken (JP)**
 • **Noguchi, Jinko, c/o Kao corp. Research Lab.**
 **Tochigi-ken (JP)**
 • **Tanaka, Masahito, c/o Kao corp. Research Lab.**
 **Tochigi-ken (JP)**
 • **Hamajima, Mitsugu, c/o Kao corp. Research Lab.**
 **Tochigi-ken (JP)**
 • **Nakanishi, Minoru, c/o Kao corp. Research Lab.**
 **Tochigi-ken (JP)**

(74) Representative: **Grünecker, Kinkeldey,**
**Stockmair & Schwanhäusser Anwaltssozietät**
**Maximilianstrasse 58**
**80538 München (DE)**

(56) References cited:
**EP-A- 0 572 033      US-A- 4 195 634**
**US-A- 5 518 761**

**Description**

BACKGROUND OF THE INVENTION

Field of the Invention:

The present invention relates to an absorbent article such as a sanitary napkin and a disposable diaper. More particularly, it relates to an absorbent article which is prevented from being distorted while worn, has an improved fit to a wearer's body and exhibits high absorptivity.

Description of Related Art:

Conventional techniques relating to absorbent articles having an elastic member include Japanese Patent Laid-Open Nos. 59-225058, 6-237956, 6-54879, 2-11138, and 6-319769.

However, the conventional techniques have their several disadvantages as follows.

In a sanitary napkin disclosed in Japanese Patent Laid-Open No. 59-225058, body fluid tends to leak in case a large amount of liquid is released thereon because of insufficient absorptivity of the absorbent member. Since the adhesion of the absorbent member and the leakproof member (elastic member) is insufficient, the former tends to twist apart from the latter, resulting in reduced absorptivity and liquid leakage.

In a sanitary napkin disclosed in Japanese Patent Laid-Open No. 6-237956, the thermoplastic fibers are dispersed in the absorbent member, so that a continuous layer of the thermoplastic fibers over the whole absorbent member result in insufficient elasticity. The presence of the dispersed thermoplastic fibers reduces the rate or capacity of absorption of the absorbent member. Supposing the thermoplastic fibers form a continuous layer, such a continuous layer will cut up a comminuted pulp layer contained in the absorbent member, resulting in hindrance to liquid permeation and diffusion within the absorbent member.

In a sanitary napkin disclosed in Japanese Patent Laid-Open Nos. 6-54879 and 2-11138, the adhesion between an absorbent member and an elastic layer or between an absorbing core and a deformable element of the sanitary napkin is insufficient so that the elasticity of the elastic layer or the deformable element cannot be fully exhibited.

An underwear liner proposed in Japanese Patent Laid-Open No. 6-319769 has poor absorptivity because a baffle made of a foamed material lacks liquid absorbing properties. In addition, the adhesion between the baffle and the absorbent member is not enough to exhibit a sufficient leakproof effect.

US-A-4,195,634 describes a sanitary napkin comprising an absorbent core, a liquid pervious material surrounding said core, a sheet of a liquid impermeable material, a plurality of track adhesive means for securing said napkin to an undergarment and stiffener means. The material of the stiffner means has a high enough level of resiliency and stiffness to resist side compressive of the napkin to a sufficient degree to aid in retaining the adhesive means in place on the undergarment when compressive forces are applied to the napkin during use.

SUMMARY OF THE INVENTION

An object of the present invention is to provide an absorbent article which is prevented from being distorted or twisted while worn, has an improved fit to a wearer's body, and exhibits high absorptivity.

As a result of extensive investigation, the present inventors have found that the above object is accomplished by an absorbent article having an absorbent body comprising an absorbent member having a specific structure and an elastic member having specific physical properties.

In a preferred embodiment the absorbent article has an absorbent body which is formed by joining an absorbent member and an elastic member into a unitary body by a specific uniting means.

The present invention has been completed based on the above findings. That is, the above object is realized by providing an absorbent article comprising a liquid-permeable surface to be brought into contact with the skin, a liquid-impermeable surface not to be brought into contact with the skin, and a liquid retentive absorbent body interposed between the liquid-permeable surface and the liquid-impermeable surface, the absorbent body comprising at least an absorbent member and an elastic member, the absorbent member comprising at least a fiber aggregate and a superabsorbent polymer, the fiber aggregate comprising the same fiber or different fibers, the elastic member satisfying the following conditions (1) and (2), and comprising thermally fusible fibers as a major component (hereinafter referred to as an absorbent article of the first invention).

(1) a fiber orientation ratio of the elastic member is less than 5.0 and

(2) a bending stiffness of the elastic member is of 4.9 x $10^{-4}$ to 1.9 $\times$ $10^{-3}$ N•cm (0.05 to 2.0 gf•cm)

In a preferred embodiment the absorbent body (4) is formed by a first uniting means of covering the elastic member (7) with the absorbent member (6) or covering the absorbent member (6) with the elastic member (7) and a second uniting means for joining the absorbent member (6) and the elastic member (7) together with an adhesive or by making a groove by pressing.

According to the absorbent article of the present invention, because distortion of an absorbent article is prevented as well as fit to a wearer's body is improved, comfortable feeling while worn is given to the wearer for a prolonged period of time. In addition, because the absorbent article of the present invention gives no leakage from the sides and keeps a close fit to a wearer's body, high absorptivity can be ensured.

BRIEF DESCRIPTION OF THE DRAWINGS

Fig. 1 is a perspective view of a sanitary napkin as an embodiment of the absorbent article.

Fig. 2 is a cross section taken along line A-A of Fig. 1.

Fig. 3 is a transverse cross section of the absorbent body of a sanitary napkin as a embodiment of the absorbent article.

Fig. 4 is a transverse cross section of the absorbent body of a sanitary napkin as a embodiment of the absorbent article. (corresponding to Fig. 3).

DESCRIPTION OF THE EMBODIMENT

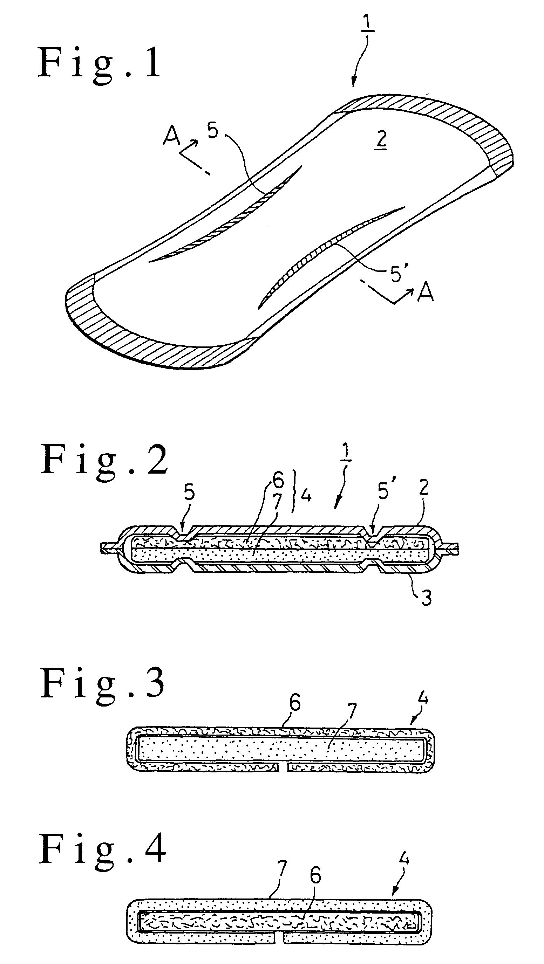

A preferred embodiment of the absorbent article is described taking a sanitary napkin as an instance with reference to drawings. Fig. 1 is a perspective view of a sanitary napkin as an embodiment of the absorbent article, and Fig. 2 is a cross section taken along line A-A of Fig. 1.

The sanitary napkin 1 shown in Figs. 1 and 2 has an elongate shape and comprises a top sheet 2 as a liquid-permeable contact surface, a back sheet 3 as a liquid-impermeable noncontact surface, and a liquid retentive absorbent body 4 interposed between the top sheet 2 and the back sheet 3.

The top sheet 2 and the back sheet 3 are of approximately the same size, and extended outwardly from the respective peripheries of the absorbent body 4, i.e., the opposing sides in the lateral direction and the opposing ends in the longitudinal direction of the absorbent body 4. The top sheet 2 and the back sheet 3 are bonded together by means of, for example, heat sealing at the extended portions.

In the sanitary napkin 1, as shown in Figs. 1 and 2, the top sheet 2, the back sheet 3, and the absorbent body 4 are partly pressed under heat to form a pair of grooves 5 and 5' in widthwise side portions. The grooves 5, 5' are curved toward the center line along the longitudinal direction of the sanitary napkin 1.

The top sheet 2 is a liquid-permeable sheet having an underwear-like touch, such as nonwoven fabrics made up of polyethylene (PE) fiber, polypropylene (PP) fiber, polyethylene terephthalate (PET) fiber, PET/PE conjugate fiber, etc. and porous films, e.g., porous polyethylene film.

The back sheet 3 includes a liquid-impermeable film sheet made of a thermoplastic resin, or a liquid-impermeable and vapor-permeable film sheet obtained by stretching a filler-containing thermoplastic resin sheet.

The above-mentioned structure is the same as in conventional sanitary napkins.

In the sanitary napkin according to this embodiment, the absorbent body 4 comprises at least an absorbent member 6 and an elastic member 7, the absorbent member 6 comprises at least a fiber aggregate and a superabsorbent polymer, the fiber aggregate comprises the same fiber or different fibers, the elastic member 7 satisfies the following conditions (1) and (2) and comprises thermally fusible fibers as a major component.

(1) a fiber orientation ratio of the elastic member is less than 5.0 and

(2) a bending stiffness of the elastic member is of $4.9 \times 10^{-4}$ to $1.9 \times 10^{-3}$ N•cm (0.05 to 2.0 gf•cm)

In the absorbent body 4, the absorbent member 6 and the elastic member 7 are joined into a unitary body preferably through at least two uniting means selected from the group consisting of adhesive application, groove-making pressing, embossing and thermal bonding.

In greater detail, the absorbent body 4 comprises a unitary body of the absorbent member 6 in a sheet form and the elastic member 7 in a sheet form and of approximately the same size as the absorbing member 6. The absorbent member 6 is arranged on the side of the top sheet 2, while the elastic member 7 on the side of the back sheet 3.

The ratio of the elastic member 7 to the absorbent member 6 in terms of the width is preferably 50% or higher. The elastic member 7 may be positioned in the central portion of the width direction of the absorbent member 6 or two pieces of the elastic member 7 may be positioned on the opposing lateral side portions. For prevention of distortion, it is particularly preferable that the absorbent member 6 and the elastic member 7 be of the same width.

The ratio of the elastic member 7 to the absorbent member 6 in terms of the length is preferably 50% or higher.

It is particularly preferable for these two members to be of the same length.

An adhesive is applied to almost over the entire surfaces of the absorbent member 6 and the elastic member 7 to be joined. The joint with the adhesion serves as a first uniting means for joining these two members into a unitary body.

The adhesive to be used includes hot-melt adhesives. The application mode and the amount of the adhesive can be chosen so as to maintain the unitarity of the absorbent member 6 and the elastic member 7 even against a large amount of liquid absorption or a hard-body movement.

A free choice can be made from various application modes as far as the absorbent member 6 and the elastic member 7 can be fixed over substantially the entire area. For example, slot spraying, spiral spraying, multi-row beads coating, dot coating, and the like can be used.

The application pattern of the adhesive is not limited as far as the absorbent member 6 and the elastic member 7 can be united as a whole. For example, an intermittent pattern such as multi-row beads (multiple lines), dots (spots at intervals) and multiple spirals at intervals can be used at choice as far as the patters meet the above purpose.

In order to obtain adhesive strength enough to join the absorbent member 6 and the elastic member 7 into a unitary body, the adhesive should be applied at an amount of 1 $g/m^2$ or more.

The grooves 5, 5' provided in opposing lateral side portions of the sanitary napkin 1 serve as a second uniting means for joining the absorbent member 6 and the elastic member 7 by pressing as shown in Fig. 2. The grooves 5, 5' are made by hot-pressing the absorbent member 6 and the elastic member 7 together with the top sheet 2 and the back sheet 3.

Thus the absorbent member 6 and the elastic member 7 in the absorbent body 4 are joined into a unitary body by the two uniting means, i.e., the first means by an adhesive and the second means by groove-making pressing. Therefore, even when the wearer's figure changes with a body movement while the sanitary napkin is worn, the separation of the absorbent member 6 and the elastic member 7 due to distortion or twist can be prevented, whereby the elasticity of the elastic member 7 can be taken full advantage of. As a result, the sanitary napkin has an improved fit to the wear's body and retains high absorptivity.

The absorbent member 6 constituting the absorbent body 4 is an absorbent sheet comprising at least a fiber aggregate and particles of a superabsorbent polymer, the fiber aggregate comprising the same fiber or different fibers. The superabsorbent polymer particles are dispersed in the single fiber aggregate or scattered in a sandwiched manner between two fiber aggregates in a layer or in shots (intermittently). The terminology "fiber aggregate" as used herein denotes an aggregate of fibers having a sheet form which is made up mainly of fibers, and includes ordinary paper, nonwoven fabric, and woven fabric.

The fibers constructing the fiber aggregate include natural cellulose fibers such as wood pulp, cotton pulp, and straw pulp; regenerated cellulose fibers such as rayon and cuprammonium rayon; synthetic hydrophilic fibers such as polyvinyl alcohol fibers and polyacrylonitrile fibers; and synthetic fibers having been rendered hydrophilic, such as polyethylene fibers, polypropylene fibers or polyester fibers having been rendered hydrophilic by treatment with a surface active agent.

It is preferred that the superabsorbent polymer particles absorb and retain 20 or more times its own weight of liquid and be capable of gelation on absorption. Suitable superabsorbent polymers include starch, crosslinked carboxymethyl cellulose, polyacrylic acid and its salt, and a polyacrylate graft polymer. In particular, superabsorbent polymers comprising water-insoluble and hydrophilic crosslinked polymer particles which are capable of absorbing a large quantity of liquid through an ionic osmotic pressure and retaining the absorbed liquid without leakage even under pressure. Such superabsorbent polymers are obtained by polymerizing acrylic acid, an acrylic acid alkali metal salt (e. g., sodium salt or potassium salt), etc., followed by crosslinking to make the polymers water-insoluble.

For prevention of distortion and improvement of absorptivity, it is preferred that the absorbent member 6 have a thickness of 0.3 to 5 mm. It is preferred, from the viewpoint of the absorption improvement, that the absorbent member 6 have the scattered superabsorbent polymer of 5 to 300 $g/m^2$. The total basis weight of the absorbent member 6 is preferably 21 to 500 $g/m^2$, still preferably 30 to 300 $g/m^2$, particularly preferably 50 to 200 $g/m^2$.

A preferred absorbent member is an absorbent sheet having a fibrous structure comprising a unitary body of two fiber aggregates containing the superabsorbent polymer inside the structure. More specifically, the absorbent sheet is made up of hydrophilic fibers, thermally fusible fibers or a paper strengthening agent, and the superabsorbent polymer, and the superabsorbent polymer is not present on the surface of the absorbent sheet which absorbs liquid but is dispersed inside the absorbent sheet and is adhesively fixed to the hydrophilic fiber constituting the absorbent sheet. The basis weight of the scattered superabsorbent polymer is 5 to 300 $g/m^2$, and the thickness of the absorbent sheet is 0.3 to 1.5 mm.

A particularly preferred absorbent member is an absorbent sheet having a fibrous structure made up of bulky hydrophilic fibers and thermally fusible fibers or a paper strengthening agent and superabsorbent polymer particles, and the superabsorbent polymer particles being not present on the absorbing surface of the absorbent sheet but dispersively fixed in the fibrous structure, the basis weight of the scattered superabsorbent polymer particles being 20 to

70 g/m$^2$, and the absorbent sheet having a thickness of 0.3 to 1.5 mm.

It is still preferred that the above-described absorbent sheet contain 3 to 30% by weight of thermally fusible fiber in its fibrous structure. In this absorbent sheet the thermally fusible fibers are thermally fused among themselves so that the structure can be maintained stably when the sheet absorbs liquid. Where the thermally fusible fiber is thermally bonded with the elastic member 7, stronger bonding force is exerted to join them into one body.

Fibers that melt on heating to bond to each other are used as the thermally fusible fibers. Examples of the thermally fusible fibers include polyolefin fibers such as polyethylene (PE), polypropylene (PP) and polyvinyl alcohol (PVA), polyester (PEs) fibers, PE/PP conjugate fibers, PE/PEs conjugate fibers, low-melting PEs/PEs conjugate fibers, PVA/PP conjugate fibers having a hydrophilic surface, and PVA/PEs conjugate fibers. The conjugate fibers may be either of a core/sheath type or a side-by-side type. These thermally fusible fibers may be used either individually or as a mixture of two or more. PVA fibers which dissolve in hot water and core/sheath type PEs fibers are preferred thermally fusible fibers for use in the present invention.

The elastic member 7 constituting the absorbent body 4 comprises a material which is deformable (e.g., curved or compressed) by outer force and capable of substantially restoring its original shape when relieved of the outer force. The elastic member 7 should satisfy the aforesaid conditions (1) and (2) and consist mainly of thermally fusible fibers.

The condition (1) is to define that the fibers in the elastic member 7 should have an orientation ratio of less than 5.0. The smaller the fiber orientation ratio, the lesser the degree of orientation of the fibers in one direction. The least fiber orientation ratio is 1.0, which means the individual fibers are oriented in every direction. At fiber orientation ratio of less than 5.0, the fibers in the elastic member 7 are oriented in random directions, exhibiting a small degree of orientation, so that the elastic member 7 is deformable in conformity with every wearing style. As a result, a sanitary napkin which undergoes no distortion and therefore exhibits stable absorptivity can be obtained. The fiber orientation ratio of the elastic member 7 is preferably 1.0 to 3.0, still preferably 1.0 to 2.0.

The fiber orientation ratio is calculated from the transmission ratio of electromagnetic waves irradiated both in the longitudinal direction and the width direction of the plane of the elastic member 7. The details of the measuring method will be described in Examples hereinafter given.

The condition (2) is to define that the elastic member 7 should have a bending stiffness of $4.9 \times 10^{-4}$ to $1.9 \times 10^{-3}$ N•cm (0.05 to 2.0 gf · cm). If the bending stiffness is less than $4.9 \times 10^{-4}$ N•cm (0.05 gf · cm), the effect of the elastic member 7 in preventing distortion is insubstantial. If it exceeds $1.9 \times 10^{-3}$ N•cm (2.0 gf · cm), the restoring force is too strong, giving discomfort to a wearer. The bending stiffness is preferably $9.8 \times 10^{-4}$ to $9.8 \times 10^{-3}$ N•cm (0.10 to 1.0 gf · cm). The details of the method for measuring the bending stiffness will be described in Examples hereinafter given.

The elastic member 7 is not particularly limited in kind as long as the above requirements are met. It is particularly preferred that the elastic member 7 be formed of nonwoven fabric consisting mainly of the above-described thermally fusible fiber.

The thermally fusible fibers for use in the elastic member 7 include various kinds of thermoplastic fibers. Examples of useful thermally fusible fibers include polyolefin fibers such as polyethylene (PE) and polypropylene (PP), polyester (PEs) fibers, such as polyethylene terephthalate (PET), PE/PP conjugate fibers, PE/PEs conjugate fibers, PP/PEs conjugated fibers, low-melting PEs/PEs conjugate fibers, and low-melting PP/PP conjugate fibers (especially core/sheath type conjugated fibers). The conjugate fibers may be either of a core/sheath type or a side-by-side type. These thermally fusible fibers may be used either individually or as a mixture of two or more thereof.

While the thermally fusible fibers can be used in the form of either short fibers or filaments of long fibers, they are preferably used in the form of short fibers so that the fibers may be oriented in all directions at random for the purpose of enhancing the deformability and restoring properties of the elastic member 7. It is still preferred that the short fibers have a fiber length of not longer than 30 mm, particularly of from 2 to 10 mm for further enhancing the deformability and restoring properties.

For the above-described purpose, it is preferred that the individual thermally fusible fibers have at least a shape bending in their longitudinal direction, that is, at least two dimensionally crimping. It is particularly preferred that the fibers have at least a shape bending in their longitudinal direction to provide a three-dimensional shape, i.e., to be crimped in three dimensions.

The thermally fusible fibers preferably have a fineness of not more than 15 deniers, particularly 1 to 10 deniers, because too thick and thereby too stiff fibers are likely to make the resulting elastic member hard and subject to buckling.

As described above, the elastic member 7 is made predominantly of thermally fusible fibers. The expression "made predominantly of thermally fusible fibers" as used here means that the elastic member 7 contains fibers of thermally fusiblity enough to exhibit its elasticity. Thus, the elastic member 7 may consist solely of the thermally fusible fibers or may contain other materials in addition to the thermally fusible fibers as long as the above-mentioned requirements are satisfied. Usable other materials include natural cellulose fibers, regenerated cellulose fibers and hydrophilic synthetic fibers. These materials are preferably incorporated in a proportion of 5 to 60% by weight based on the weight of the elastic member 7.

It is preferable that the thermally fusible fibers used in the elastic member 7 be subjected to treatment for rendering hydrophilic with a surface active agent and the like either before or after being formed into an elastic member. In this case, the elastic member 7 can work as a part of an absorbent member, resulting in further improved absorptivity.

The method for preparing the nonwoven fabric comprising the thermally fusible fiber is not particularly limited, and any conventional method can be used. Where the thermally fusible fibers are short fibers, an air-laid method or a suction heat bonding method of a carding web are suitable for obtaining bulky nonwoven fabric having a three-dimensional network structure.

The thickness of the elastic member 7 is preferably 0.1 to 7.0 mm, still preferably 0.5 to 5 mm. If the thickness is less than 0.1 mm, the elastic member may have insufficient restoring force. If the thickness exceeds 7 mm, the resulting absorbent article becomes too thick for use as a sanitary napkin, giving discomfort to a wearer while worn. Accordingly, the above range is preferred. When using nonwoven fabric as the elastic member 7, the term "thickness" as referred to above means the thickness under a load of 0.5 g/cm$^2$.

The basis weight of the elastic member 7 is preferably 5 to 100 g/m$^2$, still preferably 15 to 80 g/m$^2$. If it is less than 5 g/m$^2$, the elastic member 7 may fail to have sufficient restoring force. If it exceeds 100 g/m$^2$, a considerable outer force would be required for deformation, giving discomfort to a wearer while worn. Accordingly, the above-specified range is preferred.

It is preferred for the elastic member 7 to have a bulk restoring force (recovering force at 25% deformation after 75% compression) of 2 to 100 g, particularly 10 to 50 g, while wet. If the bulk restoring force while wet is less than 2 g, the sanitary napkin has an insufficient distortion preventive effect. If it exceeds 100 g, discomfort is given to a wearer while the sanitary napkin is being worn, due to the too strong restoring force. Accordingly, the above range is preferred.

The bulk restoring force can be determined as follows.

A sample of 40.0 mm in width and 170 mm in length is cut out of the elastic member. Ten grams of water is scattered over the whole of the sample. The opposing ends in the longitudinal direction of the sample are joined with an overlap of 2 mm and stapled at two points to make a cylindrical specimen having a height of 40.0 mm. The specimen was placed on a stand of Tensilon RTM-25 (manufactured by Toyo Baldwin K.K.) such that one of the longer sides of the sample is positioned at the lower side. The specimen was 75% compressed in a compression test mode at a rate of 10 mm/min and then let to restore. The force exerted for recovering to 25% deformation was measured and taken as a bulk restoring force while wet.

In the production of the sanitary napkin shown in Figs. 1 and 2, the uniting means for joining the absorbent member 6 and the elastic member 7 is not limited to a combination of the joint with an adhesive and the groove-making pressing as described above, and other combinations of uniting means can be used.

That is, at least two uniting means can be selected from the group consisting of adhesive application, groove-making pressing, embossing and thermal bonding. In particular, a combination of a uniting means selected from the group consisting of adhesive application, embossing and thermal bonding and a uniting means by groove-making pressing is used. More specifically, a combination of groove-making pressing and embossing (in this case the embossing is applied to a part or the entire surfaces of the absorbent member 6 and the elastic member 7) and a combination of groove-making pressing and thermal bonding (in this case thermal bonding is conducted in a part or the whole surfaces of the absorbent member 6 and the elastic member 7) can be used suitably.

A preferred embodiment of the absorbent article will be illustrated with reference to a sanitary napkin as an instance by referring to the accompanying drawings.

Fig. 3 is a transverse cross section of the absorbent body of a sanitary napkin as a embodiment of the absorbent article. Fig. 4 is a widthwise cross section of the absorbent body of a sanitary napkin as a embodiment of the absorbent article (corresponding to Fig. 3).

The members shown in Figs. 3 and 4 are denoted by the same reference numerals as used in Figs. I and 2.

Similarly to the sanitary napkin shown in Figs. 1 and 2, the absorbent body 4 of the sanitary napkin shown in Fig. 3 comprises an absorbent member 6 in a sheet form and an elastic member 7 in a sheet form, but these two members are different in size. The absorbent member 6 and the elastic member 7 are joined into a unitary body by using at least two uniting means. The first uniting means consists of covering all of the upper and lower surfaces and opposing lateral sides of the elastic member 7 with the absorbent member 6. The second uniting means is either joining the absorbent member 6 and the elastic member 7 by applying an adhesive to substantially the entire surfaces to be joined of the two members or by groove-making pressing. Fig. 3 shows an embodiment in which the two members are joined together with an adhesive as a second uniting means.

Thus, the absorbent member 6 and the elastic member 7 are joined by at least two uniting means into the absorbent body 4. Therefore, similarly to the sanitary napkin shown in Figs. 1 and 2, the separation of the absorbent member 6 and the elastic member 7 due to distortion is prevented even when the wearer's figure changes with a body movement while the sanitary napkin is being worn, whereby the elasticity of the elastic member 7 can be fully exhibited.

As a result, the sanitary napkin has an improved fit to a wear's body and retains high absorptivity.

While in Fig. 3 the absorbent member 6 covers entire periphery of the elastic member 7, not all the periphery of the elastic member 7 needs to be covered by the absorbent member 6.

The range of the amount of the adhesive used in the absorbent body 4 of Fig. 3 is substantially the same as that used in the absorbent body of the sanitary napkin shown in Figs. 1 and 2 or rather lower than that for the following reason. Provided that the absorbent body 4 shown in Fig. 3 and that shown in Figs. 1 and 2 have the same size, the joint surface area between the absorbent member 6 and the elastic member 7 in the former is about twice as large as that of the latter. Therefore, the reduced adhesion due to the smaller amount of the adhesive can be compensated for by an increase in joint surface area.

In the absorbent body 4 of Fig. 3, it is preferred that the elastic member 7 covered with the absorbent member 6 be formed from a hydrophilic material or made hydrophilic by an appropriate treatment for the following reason. When liquid passes through the absorbent member 6 covering the upper surface of the elastic member 7 and reaches the elastic member 7, if the elastic member 7 is hydrophilic, the liquid can pass therethrough and reach the absorbent member 6 covering the lower surface of the elastic member 7. It follows that the liquid can be absorbed by the lower part of the absorbent member 6 underneath the elastic member 7, i.e., the absorption capacity increases.

The absorbent body 4 of the sanitary napkin shown in Fig. 4 comprises an absorbent member 6 and an elastic member 7, which are joined into a unitary body with an adhesive, as the second uniting means, applied to the facing surfaces of the two members. This construction is the same as the embodiment shown in Fig. 3. The difference between the absorbent body 4 shown in Fig. 4 and that shown in Fig. 3 lies in that the relation between a covering member and a covered member, which is the first joining means, is reversed.

That is, in the absorbent body 4 of Fig. 4, all of the upper and lower surfaces and opposing lateral sides of the absorbent member 6 are covered with the elastic member 7 to give a first uniting means.

Similarly here in the absorbent body 4 shown in Fig. 4, the absorbent member 6 and the elastic member 7 are joined by at least two uniting means so that the same advantageous effects of the absorbent body of the sanitary napkin shown in Figs. 1 and 2 and that shown in Fig. 3 are exhibited.

While in Fig. 4 the elastic member 7 covers the entire surface of the absorbent member 6, not all the surface of the absorbent member 6 needs to be covered by the elastic member 7.

In the absorbent body 4 shown in Fig. 4, it is preferred that the elastic member 7 covering the absorbent member 6 be formed from a hydrophilic material or made hydrophilic by an appropriate treatment so that liquid may pass from the elastic member 7 to the absorbent member 6 smoothly. In this case, either only the part of the elastic member 7 which covers the absorbent member 6 or the whole of the elastic member 7 can be formed of a hydrophilic material or made hydrophilic.

While the absorbent articles according to the present invention have been illustrated with reference to their preferred embodiments, the absorbent articles of the present invention are by no means limited thereto, and various changes and modifications can be made therein without departing from the scope of the present invention.

The absorbing article of the present invention is applicable to not only sanitary napkins as hereinabove described but nursing pads, underwear liners, disposable diapers, incontinent pads.

The effectiveness of the present invention will now be demonstrated by way of Examples.

EXAMPLE 1

An absorbing sheet having a total basis weight of 110 g/m$^2$ and a thickness of 1 mm was used as an absorbent member. The absorbing sheet comprised a fiber aggregate having a basis weight of 80 g/m$^2$, made up of 97 parts by weight of crosslinked pulp ("HBA" (trade name) produced by Weyerhauser Paper Co., Ltd.); obtained by treating wood pulp with a crosslinking agent and 3 parts by weight of polyvinyl alcohol binder fiber ("Fibribond" (trade name) of Sansho K.K.), having 30 g/m$^2$ of superabsorbent polymer particles incorporated therein.

Three-dimensionally crimped PET/PE core/sheath type conjugate fibers having a fineness of 3 deniers and a length of 5 mm were formed into a web by an air-laid method, followed by thermal bonding to obtain nonwoven fabric having a basis weight of 30 g/m$^2$ and a thickness of 1 mm. The resulting nonwoven fabric was used as an elastic member.

A hot-melt adhesive was applied spirally between the absorbent member and the elastic member at a basis weight of 10 g/m$^2$ over a width of 40 mm, and the absorbent member and the elastic member were adhered to prepare an absorbent body.

The absorbent body was combined with suction heat-bonded nonwoven fabric to be a contact surface and a moisture-permeable sheet of polyethylene to be a noncontact surface, and the combination was joined into a one body by groove-making pressing as shown in Figs. 1 and 2 to prepare a sanitary napkin having the structure shown in Fig. 2.

EXAMPLE 2

An absorbing sheet having a total basis weight of 100 g/m$^2$ and a thickness of 0.8 mm was produced for use as an absorbent member in the same manner as in Example 1, except for changing the amount of the incorporated superabsorbent polymer particles to 20 g/m$^2$.

Three-dimensionally crimped PET/PE core/sheath type conjugate fibers having a fineness of 4 deniers and a length of 5 mm were formed into a web by an air-laid method, followed by thermal bonding to obtain nonwoven fabric having a basis weight of 40 g/m$^2$ and a thickness of 1.2 mm. The resulting nonwoven fabric was used as an elastic member.

A sanitary napkin having the structure shown in Fig. 2 was prepared in the same manner as in Example 1, except for using the above-described absorbent member and the elastic member.

EXAMPLE 3

Three-dimensionally crimped PP/PE core/sheath type conjugate fibers having a fineness of 1.5 deniers and a length of 7 mm were formed into a web by an air-laid method, followed by thermal bonding to obtain nonwoven fabric having a basis weight of 30 g/m$^2$ and a thickness of 0.7 mm. The resulting nonwoven fabric was used as an elastic member.

The absorbent member used in Example 2 was covered with the above nonwoven fabric. The facing surfaces of the two members were adhered by applying a hot-melt adhesive in spiral at a basis weight of 10 g/m$^2$ with a width of 40 mm to obtain an absorbent body having the structure shown in Fig. 4.

A sanitary napkin was prepared in the same manner as in Example 1, except for using the above-described absorbent body.

COMPARATIVE EXAMPLE 1

An absorbing sheet having a total basis weight of 110 g/m$^2$ and a thickness of 1 mm comprising a fiber aggregate made up of wood pulp ("NB-420" (trade name) produced by Weyerhauser Paper Co., Ltd.), having a basis weight of 80 g/m$^2$ and containing therein 30 g/m$^2$ of superabsorbent polymer particles was used as an absorbent member.

Non-crimped PET/PE core/sheath type conjugate fibers having a fineness of 1.5 deniers and a length of 51 mm were formed into a web by carding, followed by thermal bonding to obtain nonwoven fabric having a basis weight of 20 g/m$^2$ and a thickness of 0.4 mm. The resulting nonwoven fabric was used as an elastic member.

A sanitary napkin was prepared in the same manner as in Example 1, except for using the above-described absorbent member and elastic member.

Evaluation of Performance

The fiber orientation ratio and bending stiffness of the nonwoven fabric used as an elastic member in Examples and Comparative Example were measured in accordance with the following methods. The results obtained are shown in Table 1 below along with the method of production, basis weight, and thickness under a load of 0.5 g/cm$^2$ of the nonwoven fabric.

In order to evaluate the performance of the sanitary napkins obtained in Examples and Comparative Example, a distortion ratio was determined, and tendency to distortion in actual use was evaluated as follows. The results obtained are also shown in Table 1.

1) Fiber Orientation Ratio

Measured with a molecular orientation analyzer "MOA-2001A" (trade name) manufactured by KS Systems K.K.

2) Bending Stiffness

The nonwoven fabric was cut into a test specimen of 20 cm long and 10 cm wide. A bending moment for a bending curvature was measured with "KES-FB2" (trade name) manufactured by Katotech, Inc.

Both longer sides of the specimen were fixed to the chucks (chuck distance: about 1 cm) and bent at a rate of curvature increase (rate of deformation in bending) of 0.5 cm/sec up to a curvature of 2.5 cm$^{-1}$ and then restored to a curvature of 1.5 cm$^{-1}$. At this time, the bending moment was measured and taken as a bending stiffness. In using a specimen whose longer side was shorter than 20 cm, the measured bending stiffness was corrected to 20 cm length.

3) Distortion Ratio

Each of the sanitary napkins obtained in Examples and Comparative Example, into which 8 g of defibrinated horse blood had previously been poured, was applied to a movable model of a female human body. The model was made to take a walking movement for 60 minutes. The napkin was removed with care, and its least width was measured. The distortion ratio was calculated from the measured width and the width before the test according to the following equation. Measurement was repeated 5 times to obtain an average. As a guide to judgement, sanitary napkins that are hardly distorted in actual use frequently exhibit not higher than 25% distortion ratios in this test, whereas those which are easily distorted frequently exhibit 30% or higher distortion ratios.

Distortion ratio (%) = (Width of napkin before test - Width of napkin after test)/Width of napkin before test x 100 (%)

4) Evaluation in Actual Use (Tendency to Distortion)

Each sanitary napkin was worn by 35 female panelists for 2 hours, and the feeling of the napkin's being distorted while worn was evaluated by choosing, from the following points 1 to 5, the expression which they thought the closest to what they felt.

1 ... Hardly distorted.
2 ... Rather hardly distorted.
3 ... Hard to say which.
4 ... Rather easily distorted.
5 ... Easily distorted.

The average point of evaluation was taken as a measure of the tendency to distortion. The smaller the point (the closer to 1), the lower the tendency to distortion. The larger the point (the closer to 5), the higher the tendency to distortion.

TABLE 1

| | Elastic Member | | | | | Napkin Performance | |
| Uniting Means | Fiber Orientation Ratio | Bending Stiffness [(gf · cm)] | Basis Weight (g/m²) | Thickness (mm) | Fiber Length (mm) | Distortion Ratio (%) | Evaluation in actual use (Tendency to Distortion) |
|---|---|---|---|---|---|---|---|
| Example 1 | Spiral HM* adhesion + groove-making pressing | 1.1 | [0.5] $4.9 \times 10^{-3}$ | 30 | 1 | 5 | 15 | 1.4 |
| Example 2 | Spiral HM adhesion + groove-making pressing | 1.1 | [0.9] $8.8 \times 10^{-3}$ | 40 | 1.2 | 5 | 10 | 1.2 |
| Example 3 | Spiral HM adhesion + covering | 2.3 | [0.3] $2.9 \times 10^{-3}$ | 30 | 0.7 | 7 | 7 | 1.1 |
| Comparative Example 1 | Spiral HM adhesion + groove-making pressing | 5.1 | [0.03] $2.9 \times 10^{-4}$ | 20 | 0.4 | 51 | 37 | 4.2 |

* Note: "HM" stands for hot-melt adhesive

As is apparent from the results in Table 1, the sanitary napkins of Examples according to the present invention have smaller distortion ratios and are less distortable in actual use as compared with the comparative sanitary napkin.

**Claims**

1. An absorbent article comprising a liquid-permeable surface to be brought into contact with the skin, a liquid-impermeable surface not to be brought into contact with the skin, and a liquid retentive absorbent body (4) interposed between said liquid-permeable surface and said liquid-imperable surface, said absorbent body (4) comprising at least an absorbent member (6) and an elastic member (7), said absorbent member (6) comprising at least a fiber aggregate and a superabsorbent polymer, said fiber aggregate comprising the same fiber or different fibers, **characterized in that** said elastic member (7) satisfying the following conditions (1) and (2), and comprising thermally fusible fibers as a major component:

   (1) a fiber orientation ratio of said elastic members is less than 5.0 and

   (2) a bending stiffness of said elastic member is of $4.9 \times 10^{-4}$ N • cm to $1.9 \times 10^{-3}$ N • cm (0.05 to 2.0 gf • cm).

2. The absorbent article according to claim 1, wherein said absorbent member (6) and said elastic member (7) are joined into a unitary body by at least two joining means selected from the group consisting of adhesive application, groove-making pressing, embossing and themal bonding.

3. The absorbent article according to calim 1, wherein said elastic member (7) comprises nonwoven fabric comprising the thermally fusible fibers as a major component.

4. The absorbent article according to claim 1, wherein said elastic member has a basis weight of 5 to 100 g/m$^2$.

5. The absorbent article according to claim 3, wherein said thermally fusible fibers which form said nonwoven fabric are short fibers having a length of 30 mm or shorter.

6. The absorbent article according to claim 3, wherein said thermally fusible fibers which form said nonwoven fabric individually have at least a bend in its longitudinal direction.

7. The absorbent article according to claim 1, wherein said absorbent member contains thermally fusible fibers.

8. The absorbent article according to claim 1, wherein said absorbent body (4) is formed by a first uniting means of covering said elastic member (7) with said absorbent member (6) or covering said absorbent member (6) with said elastic member (7) and a second uniting means for joining said absorbent member (6) and said elastic member (7) together with an adhesive or by making a groove by pressing.

9. The absorbent article according to claim 8, wherein said absorbent body (4) is formed by a first uniting means substantially covering the entire surface of said elastic member (7) with said absorbent member (6) or substantially covering the entire surface of said absorbent member (6) with said elastic member (7).

**Patentansprüche**

1. Absorptionsfähiger Gegenstand, der umfasst eine für Flüssigkeit durchlässige Oberfläche, die mit der Haut in Kontakt gebracht werden soll, eine für Flüssigkeit undurchlässige Oberfläche, die nicht mit der Haut in Kontakt gebracht werden soll, und einen Flüssigkeit zurückhaltenden absorptionsfähigen Körper (4), der zwischen der für Flüssigkeit durchlässigen Oberfläche und der für Flüssigkeit undurchlässigen Oberfläche angeordnet ist und mindestens ein absorptionsfähiges Element (6) und ein elastisches Element (7) umfasst, wobei das absorptionsfähige Element (6) mindestens ein Faseraggregat und ein superabsorptionsfähiges Polymer umfasst, wobei das Faseraggregat die gleichen Fasern oder unterschiedliche Fasern umfasst, **dadurch gekennzeichnet, dass** das elastische Element (7) den folgenden Bedingungen (1) und (2) genügt und als eine Hauptkomponente thermisch schmelzbare Fasern aufweist:

   (1) das Faserorientierungs-Verhältnis der elastischen Elemente beträgt weniger als 5,0 und

   (2) die Biegesteifheit des elastischen Elements beträgt $4,9 \times 10^{-4}$ N.cm bis $1,9 \times 10^{-3}$ N.cm (0,05 - 2,0 gf.cm).

2. Absorptionsfähiger Gegenstand nach Anspruch 1, in dem das absorptionsfähige Element (6) und das elastische Element (7) durch mindestens zwei Verbindungsmaßnahmen, ausgewählt aus der Gruppe, die besteht aus dem Auftragen von Klebstoff, dem Erzeugen von Nuten durch Pressen, dem Prägen und dem thermischen Verbinden, zu einem einheitlichen Körper miteinander verbunden worden sind.

3. Absorptionsfähiger Gegenstand nach Anspruch 1, in dem das elastische Element (7) einen Vliesstoff umfasst, der als eine Hauptkomponente thermisch schmelzbare Fasern umfasst.

4. Absorptionsfähiger Gegenstand nach Anspruch 1, in dem das elastische Element ein Flächengewicht von 5 bis 100 g/m$^2$ aufweist.

5. Absorptionsfähiger Gegenstand nach Anspruch 3, in dem die thermisch schmelzbaren Fasern, die den Vliesstoff bilden, kurze Fasern mit einer Länge von 30 mm oder kürzer sind.

6. Absorptionsfähiger Gegenstand nach Anspruch 3, in dem die thermisch schmelzbaren Fasern, die den Vliesstoff bilden, jeweils mindestens eine Biegung in ihrer Längsrichtung aufweisen.

7. Absorptionsfähiger Gegenstand nach Anspruch 1, in dem das absorptionsfähige Element thermisch schmelzbare Fasern enthält.

8. Absorptionsfähiger Gegenstand nach Anspruch 1, in dem der absorptionsfähige Körper (4) gebildet worden ist durch eine erste Verbindungsmaßnahme, die das elastische Element (7) mit dem absorptionsfähigen Element (6) bedeckt oder das absorptionsfähige Element (6) mit dem elastischen Element (7) bedeckt, und eine zweite Verbindungsmaßnahme zum Verbinden des absorptionsfähigen Elements (6) mit dem elastischen Element (7) mittels eines Klebstoffes oder durch Erzeugung einer Nut durch Pressen.

9. Absorptionsfähiger Gegenstand nach Anspruch 8, in dem der absorptionsfähige Körper (4) gebildet worden ist durch eine erste Vereinigungsmaßnahme, die praktisch die gesamte Oberfläche des elastischen Elements (7) mit dem absorptionsfähigen Element (6) bedeckt oder praktisch die gesamte Oberfläche des absorptionsfähigen Elements (6) mit dem elastischen Element (7) bedeckt.

**Revendications**

1. Article absorbant comprenant une surface perméable au liquide destinée à être amenée en contact avec la peau, une surface imperméable au liquide destinée à ne pas être amenée en contact avec la peau, et un corps absorbant étanche au liquide (4) intercalé entre ladite surface perméable au liquide et ladite surface imperméable au liquide, ledit corps absorbant (4) comprenant au moins un élément absorbant (6) et un élément élastique (7), ledit élément absorbant (6) comprenant au moins un agrégat fibreux et un polymère superabsorbant, ledit agrégat fibreux comprenant les mêmes fibres ou des fibres différentes, **caractérisé en ce que** ledit élément élastique (7) satisfait aux conditions suivantes (1) et (2) et comprenant des fibres thermofusibles en tant que composant principal :

(1) un rapport d'orientation des fibres dudit élément élastique est inférieur à 5,0 et
(2) une rigidité à la flexion dudit élément élastique est de 4,9 x 10$^{-4}$ N • cm à 1,9 x 10$^{-3}$ N • cm (de 0,05 à 2,0 gf • cm).

2. Article absorbant selon la revendication 1, dans lequel ledit élément absorbant (6) et ledit élément élastique (7) sont joints de manière à former un corps unitaire grâce à au moins deux moyens de jonction sélectionnés parmi le groupe constitué par l'application adhésive, le pressage pour formation de rainures, le gaufrage ou le liage thermique.

3. Article absorbant selon la revendication 1, dans lequel ledit élément élastique (7) comprend un tissu non-tissé comprenant les fibres thermofusibles en tant que composant principal.

4. Article absorbant selon la revendication 1, dans lequel ledit élément élastique présente une masse surfacique de 5 à 100 g/m$^2$.

5. Article absorbant selon la revendication 3, dans lequel lesdites fibres thermofusibles qui forment ledit tissu non-tissé sont des fibres courtes ayant une longueur de 30 mm ou moins.

**6.** Article absorbant selon la revendication 3, dans lequel lesdites fibres thermofusibles qui forment ledit tissu non-tissé présentent individuellement au moins une courbure dans leur sens longitudinal.

**7.** Article absorbant selon la revendication 1, dans lequel ledit élément absorbant contient des fibres thermofusibles.

**8.** Article absorbant selon la revendication 1, dans lequel ledit corps absorbant (4) est formé par des premiers moyens de jonction de recouvrement dudit élément élastique (7) avec ledit élément absorbant (6) ou de recouvrement dudit élément absorbant (6) avec ledit élément élastique (7) et des seconds moyens de jonction destinés à joindre ledit élément absorbant (6) et ledit élément élastique (7) l'un à l'autre à l'aide d'un adhésif ou en formant une rainure par pressage.

**9.** Article absorbant selon la revendication 8, dans lequel ledit corps absorbant (4) est formé par des premiers moyens de jonction recouvrant sensiblement toute la surface dudit élément élastique (7) avec ledit élément absorbant (6) ou recouvrant sensiblement toute la surface dudit élément absorbant (6) avec ledit élément élastique (7).

Fig.1

Fig.2

Fig.3

Fig.4